# EUROPEAN PATENT APPLICATION

(11) **EP 4 778 917 A1**
(43) Date of publication of application: **22.07.2026**
(21) Application number: 24865357.8
(22) Date of filing: 05.09.2024
(51) Int. Cl.: C07C 381/12, C07B 63/00, C07C 211/63, C07C 215/40, C07F 9/54, C07F 11/00

(54) **SOLVATE OF POLYACID SALT AND METHOD FOR PRODUCING SAME**

(30) Priority: 11.09.2023 JP 2023147024
(71) Applicant: TOKYO OHKA KOGYO CO., LTD., Kawasaki-shi, Kanagawa 211 0012 (JP)
(72) Inventor: UNO, Kakishi, Kawasaki-shi, Kanagawa 211-0012 (JP); INARI, Takatoshi, Kawasaki-shi, Kanagawa 211-0012 (JP)
(74) Representative: Plasseraud IP
(86) International application number: PCT/JP2024/031892
(87) International publication number: WO 2025/057857

(57) **Abstract**

An object of the present invention is to provide a solvate of a polyacid salt having improved dispersibility or solubility in a solvent as compared with the polyacid salt itself, wherein the solvent that solvates the polyacid salt includes a first solvent that is a good solvent for the polyacid salt, and a method for producing the solvate of the polyacid salt.

## Description

### Technical Field

The present invention relates to a solvate of polyacid salt and a method for producing it.

### Background Art

The polyacid is an anionic metal oxide cluster represented by the general formula [MₓO_{y}]ⁿ⁻ (wherein x, y, and n all denote natural numbers). The metal atom M constituting the polyacid is referred to as a polyatom and is, for example, Mo (hexavalent or pentavalent), W (hexavalent or pentavalent), V (pentavalent), Nb (pentavalent), Ta (pentavalent), or the like. The polyacid can be broadly divided into an isopolyacid composed of the polyatom M and an oxoacid, and a heteropolyacid ([X_{w}MₓO_{y}]ⁿ⁻ (wherein w, x, y, and n all denote natural numbers)) containing a different type of heteroatom X (for example, P⁵⁺, Si⁴⁺, Ge⁴⁺, B³⁺, S⁶⁺, or the like as a heteroatom X) in addition to the polyatom M and oxygen.

Isopolyacid salts and heteropolyacid salts have attracted attention as materials for optoelectronics, catalysts, and the like. If polyacid salts and heteropolyacid salts can be dispersed or dissolved in water or organic solvents at high concentrations, it can be expected that the polyacid salts and the heteropolyacid salts are effectively utilized in various industrial applications by being coated on the surfaces of various parts to form films, by being used as additive agents for catalysts, or the like.

Patent Literature 1 discloses a tantalic acid dispersion liquid containing tantalic acid and an amine in water as a novel tantalic acid dispersion liquid that does not contain a less volatile organic component and has high dispersibility in water, and it is presumed that the amine can be ionically bonded to the tantalic acid to be present in water as an ion with a polyacid structure, thereby increasing the solubility in water.

### Citation List

### Patent Literature

PTL 1: European Patent No. 4186866

### Summary of Invention

### Technical Problem

An object of the present invention is to provide a solvate of a polyacid salt having improved dispersibility or solubility in a solvent as compared with the polyacid salt itself, and a method for producing the solvate of the polyacid salt.

### Solution to Problem

As a result of extensive studies to solve the above problems, the present inventors have completed the present invention by finding that the dispersibility or solubility of a polyacid salt in a solvent can be improved by solvating the polyacid salt with the solvent including a first solvent that is a good solvent for the polyacid salt.

More specifically, the present invention relates to the following inventions.
<1> A solvate of a polyacid salt, wherein
   a solvent that solvates the polyacid salt includes a first solvent that is a good solvent for the polyacid salt.
<2> The solvate of the polyacid salt according to <1>, wherein an anion moiety of the polyacid salt is an isopolyoxomolybdate anion, an isopolyoxotungstate anion, an isopolyoxoniobate anion, an isopolyoxotantalate anion, a heteropolyoxomolybdate anion, a heteropolyoxotungstate anion, a heteropolyoxovanadate anion, a heteropolyoxoniobate anion, or a heteropolyoxotantalate anion.
<3> The solvate of the polyacid salt according to <1>, wherein a cation moiety of the polyacid salt is an onium cation.
<4> The solvate of the polyacid salt according to <3>, wherein the onium cation is an organic sulfonium cation, an organic iodonium cation, an organic quaternary ammonium cation, or an organic phosphonium cation.
<5> The solvate of the polyacid salt according to <1>, wherein the first solvent has a dipole moment (D) per van der Waals volume (Å³) of 0.055 D/Å³ or more and a hydrogen-bonding term (ΔH) in a Hansen solubility parameter of 17 MPa^{1/2} or less.
<6> The solvate of the polyacid salt according to <1>, wherein the solvent that solvates the polyacid salt further includes a second solvent (except the first solvent), the second solvent being a poor solvent for the polyacid salt.
<7> A method for producing a solvate of a polyacid salt, including dissolving the polyacid salt in one or more first solvents that are good solvents for the polyacid salt to prepare a good solvent solution.
<8> The method for producing a solvate of a polyacid salt according to <7>, further including
   adding a second solvent (except the first solvent) to the good solvent solution, the second solvent being a poor solvent for the polyacid salt.
<9> A film-forming material produced using the solvate of the polyacid salt according to <1>.

### Advantageous Effects of Invention

The present invention can provide a solvate of a polyacid salt having improved dispersibility or solubility in a solvent as compared with the polyacid salt itself, and a method for producing the solvate of the polyacid salt. A polyacid salt can also be dispersed or dissolved in a solvent at a higher concentration to be suitably used as a film-forming material. Furthermore, a solvate of a polyacid salt can expand the range of a solvent that can dissolve the polyacid salt, allows selection and use of a solvent that is not subject to national laws and regulations or user regulations (CMR substance: carcinogenic substance, mutagenic substance, or reproductive toxicant) or the like, and can enhance the industrial applicability of the polyacid salt.

### Description of Embodiments

Preferred embodiments of the present invention will be described in detail below. However, the present invention is not limited to the following embodiments.

The term "solvate", as used herein, refers to a polyacid salt to which solvent molecules are bonded by a relatively weak force, such as an electrostatic interaction, a hydrogen bond, a charge transfer bond, or a coordinate bond, and the number of bonded solvent molecules may not be constant, and a plurality of solvent molecules may participate in solvation. The solvate includes a chemically acceptable solvate and includes both a stoichiometric solvate and a non-stoichiometric solvate. Such a solvate includes both a solution phase and an isolable solvate.

The term "halogen atom", as used herein, refers to a fluorine atom, a chlorine atom, a bromine atom, or an iodine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -NH(C=O)O-, -S-, -SO-, or -SO₂- (provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -C(=O)O-, -OCO-, - CONH-, -NHCO-, -NH(C=O)O-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methyl vinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbonyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₂₋₁₈ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "C₂₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₂₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -C(=O)O-, -OCO-, -CONH-, - NHCO-, -NH(C=O)O-, -S-, or -SO₂- (provided that adjacent divalent carbon atoms are not replaced at the same time). The same applies to the "C₁₋₁₈ hydrocarbyl group" and the like used in the following description of the definition of the "C₁₋₁₈ hydrocarbyloxy group" and the like.

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₂₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "C₁₋₁₈ hydrocarbyloxy group", a divalent carbon atom at any position excluding the terminals in the "C₁₋₁₈ hydrocarbyl group" is preferably replaced by -O-, - C(=O)-, and/or -C(=O)O-, the "C₁₋₁₈ hydrocarbyloxy group" is more preferably a "C₁₋₁₈ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group".

The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonylamino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes a hydrogen atom of a carboxy group or a hydroxy group (including a phenolic hydroxy group or the like) and is dissociated by the action of an acid.

The term "acid-dissociable group" is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

The phrase "may have a substituent", as used herein, is not particularly limited provided that it is chemically acceptable and has the advantages of the present invention. The "substituent" is, for example, (1) a halogen atom, (2) a hydroxy group, (3) a thiol group, (4) a nitro group, (5) a cyano group, (6) a carboxy group, (7) an amino group, (8) a sulfo group, (9) a group in which a hydrogen atom of a carboxy group or a hydroxy group is substituted by an acid-dissociable group, or (10) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of its hydrogen atoms may be substituted by (1) to (9), a divalent carbon atom at any position excluding the terminals of the substituent may be replaced by -O-, -C(=O)-, -C(=O)O-, -OCO-, -CONH-, -NHCO-, - NH(C=O)O-, -S-, or -SO₂- (provided that adjacent divalent carbon atoms are not replaced at the same time), or the like.

### [1. Solvate of Polyacid Salt]

A solvate of a polyacid salt according to an embodiment of the present invention is characterized in that a solvent that solvates the polyacid salt includes a first solvent that is a good solvent for the polyacid salt. The "polyacid salt" and the "solvent that solvates the polyacid salt" will be described below.

### [1-1. polyacid salt]

The method for synthesizing the polyacid salt is not particularly limited and can be a known and commonly used method. The method for synthesizing the polyacid salt is, for example, a salt exchange method, an ion exchange method, or the like.

The polyacid salt is composed of an anion moiety of an isopolyacid anion or a heteropolyacid anion and a cation moiety serving as a counter cation therefor. The anion moiety and the cation moiety of the polyacid salt will be separately described below.

### [1-1-1. Anion Moiety of Polyacid Salt]

The anion moiety of the polyacid salt is composed of an isopolyacid anion or a heteropolyacid anion.

The isopolyacid anion is, for example, an isopolyoxomolybdate anion, an isopolyoxotungstate anion, an isopolyoxovanadate anion, an isopolyoxoniobate anion, an isopolyoxotantalate anion, or the like.

The isopolyoxomolybdate anion is, for example, [MoO₄]²⁻, [Mo₇O₂₄]⁶⁻, [Mo₈O₂₆]⁴⁻, or the like. The isopolyoxotungstate anion is, for example, [W₄O₁₃]²⁻, [W₅O₁₆]²⁻, [W₆O₁₉]²⁻, [W₇O₂₂]²⁻, [W₇O₂₄]⁶⁻, [H_{z}W₁₂O₄₀]^{-(8-z)} (wherein z denotes an integer in the range of 1 to 4), [W₁₀O₃₂]⁴⁻, [H₄W₁₁O₃₈]⁶⁻, [H₇W₁₁O₄₀]⁷⁻, [HW₅O₁₉]⁷⁻, [H₃W₁₁O₂₂]⁵⁻, or the like. The isopolyoxovanadate anion is, for example, [V₄O₁₂]⁴⁻, [V₁₀O₂₈]⁶⁻, or the like, the isopolyoxoniobate anion is, for example, [Nb₆O₁₉]⁸⁻, [Nb₁₀O₂₈]⁶⁻, or the like, and the isopolyoxotantalate anion is, for example, [Ta₆O₁₉]⁸⁻, [Ta₈O₂₁]²⁻, or the like.

The isopolyoxomolybdate anion, the isopolyoxotungstate anion, the isopolyoxovanadate anion, the isopolyoxoniobate anion, and the isopolyoxotantalate anion include various isomers and the like.

The heteropolyacid anion is, for example, a heteropolyoxomolybdate anion, a heteropolyoxotungstate anion, a heteropolyoxovanadate anion, a heteropolyoxoniobate anion, a heteropolyoxotantalate anion, or the like.

The heteropolyoxomolybdate anion is, for example, a phosphomolybdate anion, a silicomolybdate anion, a boromolybdate anion, a phosphotungstomolybdate anion, a cobalt molybdate anion, an arsenic molybdate anion, a germanium molybdate anion, or the like. The heteropolyoxotungstate anion is, for example, a phosphotungstate anion, a silicotungstate anion, a borotungstate anion, a cobalt tungstate anion, an arsenic tungstate anion, a germanium tungstate anion, or the like. The heteropolyoxovanadate anion is, for example, a phosphomolybdovanadate anion, a phosphomolybdotungstomolybdate anion, a boromolybdovanadate anion, a boromolybdotungstovanadate anion, or the like.

The heteropolyoxomolybdate anion, the heteropolyoxotungstate anion, the heteropolyoxovanadate anion, and the like include a Keggin type, a Dawson type, an Anderson type, and deficient species and isomers thereof.

### [1-1-2. Cation Moiety of Polyacid Salt]

The type of the salt of the polyacid salt (isopolyoxometalate or heteropolyoxometalate) may be, but is not limited to, a salt with a proton, an onium cation (for example, an organic onium cation, an organic sulfonium cation, an organic iodonium cation, an organic quaternary ammonium cation, an organic phosphonium cation, or the like), an alkali metal ion, or the like. From the perspective of improving actinic ray sensitivity or radiation sensitivity, the type of salt of the polyacid salt is preferably a salt with an onium cation, and the onium cation is preferably an onium cation having at least one unsaturated bond that may have a substituent, the unsaturated bond is, for example, a carbon-carbon double bond or a carbon-carbon triple bond, and among these, a carbon-carbon double bond is more preferred, and a benzene ring is still more preferred. The onium cation is, for example, an organic sulfonium cation, an organic iodonium cation, an organic quaternary ammonium cation, an organic phosphonium cation, or the like.

### [1-1-2-1. Organic Sulfonium Cation]

The organic sulfonium cation is not particularly limited and may be a known and commonly used organic sulfonium cation. The organic sulfonium cation is, for example, an organic sulfonium cation represented by the formula (I).

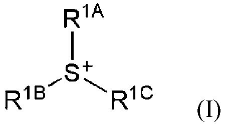

In the formula (I), R^{1A}, R^{1B}, and R^{1C} each independently denote a C₁-₁₈ hydrocarbyl group, a 3- to 18-membered non-aromatic heterocyclic group, or a 5- to 18-membered aromatic heterocyclic group,
a divalent carbon atom at any position except the end(s) of R^{1A}, R^{1B}, and R^{1C} may be replaced by -O-, -C(=O)-, -C(=O)O-, -OCO-, -CONH-, -NHCO-, -NH(C=O)O-, -S-, or - SO₂- (provided that adjacent divalent carbon atoms are not replaced at the same time),
a hydrogen atom in R^{1A}, R^{1B}, and R^{1C} may be substituted by, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a C₁-₁₈ hydrocarbyl group, a C₁-₁₈ hydrocarbyloxy group, a C₁-₁₈ hydrocarbylcarbonyl group, a C₁-₁₈ hydrocarbylcarbonyloxy group, a C₁-₁₈ hydrocarbyloxycarbonyl group, a C₁-₁₈ hydrocarbyloxycarbonyloxy group, or a C₁-₁₈ hydrocarbylthio group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time); and
any two of R^{1A}, R^{1B}, and R^{1C} may be linked to each other directly by a single bond or via a divalent linking group -O-, -S-, -C(=O)-, -S(=O)-, -S(=O)₂-, -C(=O)O-, or a C₁₋₃ alkylene group to form a ring together with a sulfur atom in the formula (I).

R^{1A}, R^{1B} and R^{1C} in the formula (I) preferably each independently denote an optionally substituted C₆₋₁₈ aryl group. A substituent for the C₆₋₁₈ aryl group is preferably a halogen atom, a haloalkyl group, a hydroxy group, a nitro group, a cyano group, an optionally substituted C₁₋₁₂ hydrocarbyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, C₁₋₁₂ hydrocarbyloxycarbonyloxy group, or a C₁-₁₈ hydrocarbylthio group, more preferably a halogen atom, a C₁₋₄ haloalkyl group, a nitro group, or a C₁₋₈ hydrocarbyloxy group.

Specific examples of the organic sulfonium cation include a dibutyl(pentyl)sulfonium cation, a triethylsulfonium cation, (2-carboxyethyl)dimethylsulfonium cation, a trimethylsulfonium cation, a dimethylphenacylsulfonium cation, a 1-(4-hydroxynaphthalen-1-yl)hexahydrothiopyrylium cation, a dimethylphenylsulfonium cation, a triphenylsulfonium cation, a tris(4-methylphenyl)sulfonium cation, a 4-methoxyphenyldiphenylsulfonium cation, a 4-iodophenyldiphenylsulfonium cation, a tris(4-fluorophenyl)sulfonium cation, a 1-phenylhexahydrothiopyrylium cation, an (ethane-1,2-diylbisoxy)bis(4,1-phenylene)bis(diphenylsulfonium) dication, a (thiodi-4,1-phenylene)bis(diphenylsulfonium) dication, a di(naphthalen-1-yl)(phenyl)sulfonium cation, a phenyl bis(2-(trifluoromethyl)phenyl)sulfonium cation, a mesityl bis(2-(trifluoromethyl)phenyl)sulfonium cation, a bis(3,5-difluorophenyl)(phenyl)sulfonium cation, a tris(3,5-difluorophenyl)sulfonium cation, a (4-(dodecanoyloxy-3,5-dimethylphenyl))diphenylsulfonium cation, a diphenyl(3-(trifluoromethoxy)phenyl)sulfonium cation, a (4-(1-adamantylcarbonyloxy)phenyl)diphenylsulfonium cation, a (4-phenylthiophenyl)diphenylsulfonium cation, a 5-phenyl-5H-thianthren-5-ium cation, a 5-(2,5-dimethylphenyl)thianthren-5-ium cation, a 5-phenyl-5H-dibenzo[b,d]thiophen-5-ium cation, a 5-(3-(trifluoromethyl)phenyl)-5H-dibenzo[b,d]thiophen-5-ium cation, a 1-(4-(t-butyl)phenyl)-1H-benzo[b]thiophen-1-ium cation, a methyldiphenylsulfonium cation, a (2-bromoethyl)diphenylsulfonium cation, a (3-chloropropyl)diphenylsulfonium cation, a benzyl(4-hydroxyphenyl)methylsulfonium cation, a (4-hydroxyphenyl)methyl(2-methylbenzyl)sulfonium cation, a 4-hydroxyphenyldimethylsulfonium cation, a diphenyl(methyl)sulfonium cation, a diphenyl(4-(phenylthio)phenyl)sulfonium cation, a dimesityl(trifluoromethyl)sulfonium cation, and a tri-p-tolylsulfonium cation.

### [1-1-2-2. Organic Iodonium Cation]

The organic iodonium cation is not particularly limited and may be a known and commonly used organic iodonium cation. The organic iodonium cation is, for example, an organic iodonium cation represented by the formula (II).

R^{2A}-I⁺-R^{2B} (II)

In the formula (II), R^{2A} and R^{2B} each independently denote a C₁-₁₈ hydrocarbyl group, a 3- to 18-membered non-aromatic heterocyclic group, or a 5- to 18-membered aromatic heterocyclic group
a divalent carbon atom at any position except the end(s) of R^{2A} and R^{2B} may be replaced by -O-, -C(=O)-, -C(=O)O-, -OCO-, -CONH-, -NHCO-, -NH(C=O)O-, -S-, or - SO₂- (provided that adjacent divalent carbon atoms are not replaced at the same time);
a hydrogen atom in R^{2A} and R^{2B} may be substituted by, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a C₁-₁₈ hydrocarbyl group, a C₁-₁₈ hydrocarbyloxy group, a C₁-₁₈ hydrocarbylcarbonyl group, a C₁-₁₈ hydrocarbylcarbonyloxy group, a C₁-₁₈ hydrocarbyloxycarbonyl group, or a C₁-₁₈ hydrocarbyloxycarbonyloxy group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, - NH(C=O)O-, -S-, or -SO₂- (provided that adjacent divalent carbon atoms are not replaced at the same time); and
furthermore, R^{2A} and R^{2B} may be linked to each other directly by a single bond or via a divalent linking group -O-, -S-, -C(=O)-, -S(=O)-, -S(=O)₂-, -C(=O)O-, or a C₁₋₃ alkylene group to form a ring together with the iodine atom in the formula (II).

R^{2A} and R^{2B} in the formula (II) preferably each independently denote an optionally substituted C₆₋₁₈ aryl group. A substituent for the C₆₋₁₈ aryl group is preferably a halogen atom, a haloalkyl group, a hydroxy group, a nitro group, a cyano group, an optionally substituted C₁₋₁₂ hydrocarbyl group, C₁₋₁₂ hydrocarbyloxy group, C₁₋₁₂ hydrocarbylcarbonyl group, C₁₋₁₂ hydrocarbylcarbonyloxy group, C₁₋₁₂ hydrocarbyloxycarbonyl group, or C₁₋₁₂ hydrocarbyloxycarbonyloxy group, preferably a halogen atom, a C₁₋₄ haloalkyl group, a nitro group, a C₁₋₈ hydrocarbyl group, or a C₁₋₈ hydrocarbyloxy group.

Specific examples of the iodonium cation include an ethynyl(phenyl)iodonium cation, a bis(pyridine)iodonium cation, a bis(2,4,6-trimethylpyridine)iodonium cation, a diphenyliodonium cation, a bis(4-(t-butyl)phenyl)iodonium cation, a (2-carboxyphenyl)(phenyl)iodonium cation, a (4-nitrophenyl)(phenyl)iodonium cation, a (3-(trifluoromethyl)phenyl)(2,4,6-trimethylphenyl)iodonium cation, a bis(4-fluorophenyl)iodonium cation, a (4-(bromomethyl)phenyl)(2,4,6-trimethoxyphenyl)iodonium cation, a 4-biphenylyl(2,4,6-trimethoxyphenyl)iodonium cation, a bis(2,4,6-trimethylphenyl)iodonium cation, a 4-isopropyl-4'-methyldiphenyliodonium cation, a (4-(trifluoromethyl)phenyl)(2,4,6-trimethylphenyl)iodonium cation, a ((4-trifluoromethyl)phenyl)(2,4,6-trimethoxyphenyl)iodonium cation, a (5-fluoro-2-nitrophenyl)(2,4,6-trimethoxyphenyl)iodonium cation, a (3-bromophenyl)(mesityl)iodonium cation, a bis(4-bromophenyl)iodonium cation, a (3,5-dichlorophenyl)(2,4,6-trimethoxyphenyl)iodonium cation, a (4-methylphenyl)(2,4,6-trimethylphenyl)iodonium cation, a (3-methylphenyl)(2,4,6-trimethylphenyl)iodonium cation, a (2-methylphenyl)(2,4,6-trimethylphenyl)iodonium cation, a phenyl(2,4,6-trimethoxyphenyl)iodonium cation, and the like.

### [1-1-2-3. Organic Quaternary Ammonium Cation]

The organic quaternary ammonium cation is not particularly limited and may be a known and commonly used organic quaternary ammonium cation. The organic quaternary ammonium cation is, for example, an organic quaternary ammonium cation represented by the formula (III).

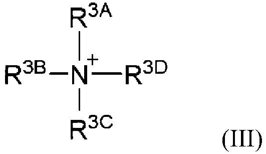

In the formula (III), R^{3A}, R^{3B}, R^{3C}, and R^{3D} each independently denote a C₁-₁₈ hydrocarbyl group, a 3- to 18-membered non-aromatic heterocyclic group, or a 5- to 18-membered aromatic heterocyclic group,
a divalent carbon atom at any position except the end(s) of R^{3A}, R^{3B}, R^{3C}, and R^{3D} may be replaced by -O-, -C(=O)-, -C(=O)O-, -OCO-, -CONH-, -NHCO-, -NH(C=O)O-, - S-, or -SO₂- (provided that adjacent divalent carbon atoms are not replaced at the same time),
a hydrogen atom in R^{3A}, R^{3B}, R^{3C}, and R^{3D} may be substituted by, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a C₁-₁₈ hydrocarbyl group, a C₁-₁₈ hydrocarbyloxy group, a C₁-₁₈ hydrocarbylcarbonyl group, a C₁-₁₈ hydrocarbylcarbonyloxy group, a C₁-₁₈ hydrocarbyloxycarbonyl group, a C₁-₁₈ hydrocarbyloxycarbonyloxy group, or a C₁-₁₈ hydrocarbylthio group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time); and
any two of R^{3A}, R^{3B}, R^{3C}, and R^{3D} may be linked to each other directly by a single bond or via a divalent linking group -O-, -S-, -C(=O)-, -S(=O)-, -S(=O)₂-, -C(=O)O-, or a C₁₋₃ alkylene group to form a ring together with a nitrogen atom in the formula (III).

R^{3A}, R^{3B}, R^{3C}, and R^{3D} in the formula (III) preferably each independently denote an optionally substituted C₁-₁₈ hydrocarbyl group, more preferably a C₁₋₁₂ alkyl group, a C₁₋₁₂ alkenyl group, a C₁₋₁₂ alkynyl group, or a C₁₋₁₂ alicyclic group.

Specific examples of the organic quaternary ammonium cation include a tetramethylammonium cation, a tetraethylammonium cation, a tetrapropylammonium cation, a tetrabutylammonium cation, a tetraheptylammonium cation, a trimethylethylammonium cation, a dimethyldiethylammonium cation, a dimethylethylpropylammonium cation, a methylethylpropylbutylammonium cation, a trimethylphenylammonium cation, a triethylhexylammonium cation, a triethylcyclohexylammonium cation, and a dodecyltrimethylammonium cation.

### [1-1-2-4. Organic Phosphonium Cation]

The organic phosphonium cation may be, but is not limited to, a known and commonly used organic phosphonium cation. The organic phosphonium cation is, for example, an organic phosphonium cation represented by the formula (IV).

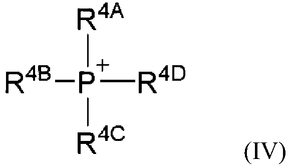

In the formula (IV), R^{4A}, R^{4B}, R^{4C}, and R^{4D} each independently denote a C₁-₁₈ hydrocarbyl group, a 3- to 18-membered non-aromatic heterocyclic group, or a 5- to 18-membered aromatic heterocyclic group,
a divalent carbon atom at any position except the end(s) of R^{4A}, R^{4B}, R^{4C}, and R^{4D} may be replaced by -O-, -C(=O)-, -C(=O)O-, -OCO-, -CONH-, -NHCO-, -NH(C=O)O-, - S-, or -SO₂- (provided that adjacent divalent carbon atoms are not replaced at the same time);
a hydrogen atom in R^{4A}, R^{4B}, R^{4C}, and R^{4D} may be substituted by, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a C₁-₁₈ hydrocarbyl group, a C₁-₁₈ hydrocarbyloxy group, a C₁-₁₈ hydrocarbylcarbonyl group, a C₁-₁₈ hydrocarbylcarbonyloxy group, a C₁-₁₈ hydrocarbyloxycarbonyl group, a C₁-₁₈ hydrocarbyloxycarbonyloxy group, or a C₁-₁₈ hydrocarbylthio group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, -NH(C=O)O-, -S-, or -SO₂- (provided that adjacent divalent carbon atoms are not replaced at the same time); and
furthermore, any two of R^{4A}, R^{4B}, R^{4C}, and R^{4D} may be linked to each other directly by a single bond or via a divalent linking group -O-, -S-, -C(=O)-, -S(=O)-, -S(=O)₂-, - C(=O)O-, or a C₁₋₃ alkylene group to form a ring together with the phosphorus atom in the formula (IV).

R^{4A}, R^{4B}, R^{4C}, and R^{4D} in the formula (IV) preferably each independently denote an optionally substituted C₁-₁₈ hydrocarbyl group, more preferably a C₁₋₁₂ alkyl group, a C₁₋₁₂ alkenyl group, a C₁₋₁₂ alkynyl group, or a C₁₋₁₂ alicyclic group.

Specific examples of the organic phosphonium cation include (methoxymethyl)triphenylphosphonium cation, tetrabutylphosphonium cation, tetraethylphosphonium cation, amyltriphenylphosphonium cation, acetonyltriphenylphosphonium cation, benzyltriphenylphosphonium cation, ethyltriphenylphosphonium cation, butyltriphenylphosphonium cation, hexyltriphenylphosphonium cation, (1-naphthylmethyl)triphenylphosphonium cation, (4-nitrobenzyl)triphenylphosphonium cation, (2-hydroxybenzyl)triphenylphosphonium cation, tetraphenylphosphonium cation, triphenyltetradecylphosphonium cation, cyclopropylphenylphosphonium cation, ethoxycarbonylmethyl(triphenyl)phosphonium cation, phenacyltriphenylphosphonium cation, and the like.

### [1-2. Solvent That Solvates Polyacid Salt]

When a solvent that solvates a polyacid salt includes a first solvent that is a good solvent for the polyacid salt, dispersibility or solubility of a solvate of the polyacid salt in an organic solvent can be improved. The solvent that solvates the polyacid salt may further include a second solvent other than the first solvent, the second solvent being a poor solvent for the polyacid salt.

### <First Solvent and Second Solvent>

The first solvent according to the present embodiment is not particularly limited provided that it is a good solvent for a predetermined polyacid salt. The first solvent is, for example, a solvent having a dipole moment (D) per van der Waals volume (Å³) of 0.055 D/Å³ or more and a hydrogen-bonding term (ΔH) in the Hansen solubility parameter of 17 MPa^{1/2} or less. The solvent that solvates the polyacid salt may include one or more of the first solvents. The solvent that solvates the polyacid salt may further include a second solvent other than the first solvent, the second solvent being a poor solvent for the polyacid salt. The second solvent is, for example, a solvent having a dipole moment (D) per van der Waals volume (Å³) of less than 0.055 D/Å³ or a hydrogen-bonding term (ΔH) in the Hansen solubility parameter of more than 17 MPa^{1/2}.

### <Dipole Moment per van der Waals Volume>

The term "van der Waals volume", as used herein, refers to the volume of a solvent molecule when atoms constituting the solvent molecule are approximated by a sphere with a van der Waals radius. In the present description, the value of alvaDesc 2.0.12 was used as the van der Waals volume of each solvent molecule.

In this description, the dipole moment of each solvent molecule was calculated using SCIGRESS FQ 3.4.4 manufactured by Fujitsu Ltd.

A dipole moment per van der Waals volume of 0.055 D/Å³ or more means that the structure of the solvent molecule is fixed and the planarity is high, or the solvent molecule has a relatively small size and polarization occurs in the solvent molecule, and it can be said that the solvent molecule can easily solvate a polyacid salt.

### <Hydrogen-Bonding Term (ΔH) in Hansen Solubility Parameter>

The Hansen solubility parameter is expressed in a three-dimensional space by dividing the solubility of a substance into three components: a dispersion term ΔD, a polarity term ΔP, and a hydrogen-bonding term ΔH. The dispersion term ΔD indicates an effect due to dispersion force, the polarity term ΔP indicates an effect due to dipole-dipole force, and the hydrogen-bonding term ΔH indicates an effect due to a hydrogen bond.

The Hansen solubility parameter can be calculated using a known and commonly used method and known and commonly used software. In the present description, ΔH as an estimated value calculated using Hansen Solubility Parameters in Practice (HSPiP) 5th Edition 5.4.02 is used.

When the hydrogen-bonding term ΔH of the Hansen solubility parameter is more than 17 MPa^{1/2}, the energy required to break a hydrogen bond between solvent molecules and solvate a polyacid salt increases, which makes it difficult to become a good solvent for the polyacid salt.

### <Specific Examples of First Solvent and Second Solvent>

The first solvent according to the present embodiment is, for example, a solvent having a dipole moment (D) per van der Waals volume (Å³) of 0.055 D/Å³ or more and a hydrogen-bonding term (ΔH) in the Hansen solubility parameter of 17 MPa^{1/2} or less, and specific examples thereof are listed in Table 1.

Table 1 shows the names of various solvents, and their van der Waals volumes (Å³), dipole moments (D), dipole moments per van der Waals volume (D/Å³), and the hydrogen-bonding terms (ΔH) in the Hansen solubility parameter.

**[Table 1]**

| No. | Solvent name | CAS No. | van der Waals volume (Å³) | Dipole moment (D) | Dipole moment per van der Waals volume (D/Å³) | ΔH |
|---|---|---|---|---|---|---|
| 1 | acetylacetone | 123-54-6 | 60.9 | 4.65 | 0.076 | 6.2 |
| 2 | acetonylacetone | 110-13-4 | 70.5 | 4.65 | 0.066 | 6.1 |
| 3 | cyclohexanone | 108-94-1 | 62 | 3.64 | 0.059 | 4.6 |
| 4 | cyclopentanone | 120-92-3 | 52 | 3.41 | 0.066 | 5.1 |
| 5 | 2-cyclohexen-1-one | 930-68-7 | 59 | 4.63 | 0.078 | 5.2 |
| 6 | 2-cyyclopenten-1-one | 930-30-3 | 49.5 | 4.11 | 0.083 | 6.2 |
| 7 | 3-methyl-2-cyclopentenone | 2758-18-1 | 69 | 5.08 | 0.074 | 6.1 |
| 8 | hydroxyacetone | 116-09-6 | 44.6 | 4.93 | 0.111 | 16.5 |
| 9 | 4-hydroxy-2-butanone | 590-90-9 | 54.2 | 4.27 | 0.079 | 14.5 |
| 10 | lactonitrile | 78-97-7 | 44.5 | 3.19 | 0.072 | 16.4 |
| 11 | 3-methoxypropionitrile | 110-67-8 | 54 | 3.55 | 0.066 | 7.0 |
| 12 | dimethylcyanamide | 1467-79-4 | 47.3 | 5.24 | 0.111 | 6.8 |
| 13 | nitromethane | 75-52-5 | 32.2 | 3.98 | 0.124 | 8.1 |
| 14 | β-propiolactone | 57-57-8 | 37.2 | 4.41 | 0.119 | 9.5 |
| 15 | 1,3,2-dioxathiolane 2-oxide | 3741-38-6 | 45.7 | 2.99 | 0.065 | 6.6 |
| 16 | 1-methyl-2-pyrrolidone | 872-50-4 | 59 | 4.46 | 0.076 | 7.4 |
| 17 | γ-butyrolactone | 96-48-0 | 46.8 | 5.09 | 0.109 | 7.8 |
| 18 | dimethyl sulfoxide | 67-68-5 | 45.1 | 4.71 | 0.104 | 8.1 |
| 19 | N,N-dimethylformamide | 68-12-2 | 47.4 | 4.57 | 0.096 | 11.4 |
| 20 | diacetone alcohol | 123-42-2 | 73.4 | 4.21 | 0.057 | 10.4 |
| 21 | acetone | 67-64-1 | 40 | 3.52 | 0.088 | 6.5 |
| 22 | propylene carbonate | 108-32-7 | 50.8 | 5.72 | 0.113 | 4.1 |
| 23 | acetonitrile | 75-05-8 | 30.9 | 3.83 | 0.124 | 6.1 |
| 24 | ethylene sulfide | 3741-38-6 | 45.7 | 2.99 | 0.065 | 6.6 |
| 25 | 3-hydroxy-3-methyl-2-butanone | 115-22-0 | 62.8 | 5.10 | 0.081 | 11.3 |

The first solvent is preferably N,N-dimethylformamide, 2-cyclopenten-1-one, 4-hydroxy-2-butanone, 3-methyl-2-cyclopentenone, acetylacetone, acetonylacetone, 2-cyclohexen-1-one, 1-methyl-2-pyrrolidone, γ-butyrolactone, cyclohexanone, cyclopentanone, diacetone alcohol, hydroxyacetone, 3-methoxypropionitrile, dimethyl cyanamide, lactonitrile, ethylene sulfide, or 3-hydroxy-3-methyl-2-butanone, more preferably N,N-dimethylformamide, 2-cyclopenten-1-one, 4-hydroxy-2-butanone, 3-methyl-2-cyclopentenone, acetylacetone, acetonylacetone, 2-cyclohexen-1-one, 1-methyl-2-pyrrolidone, or γ-butyrolactone, still more preferably N,N-dimethylformamide, 2-cyclopenten-1-one, 4-hydroxy-2-butanone, 3-methyl-2-cyclopentenone, or γ-butyrolactone.

The second solvent according to the present embodiment is, for example, a solvent having a dipole moment (D) per van der Waals volume (Å³) of less than 0.055 D/Å³ or a hydrogen-bonding term (ΔH) in the Hansen solubility parameter of more than 17 MPa^{1/2}, and specific examples thereof are listed in Table 2.
Table 2 shows the names of various solvents, and their van der Waals volumes (Å³), dipole moments (D), dipole moments per van der Waals volume (D/Å³), and the hydrogen-bonding terms (ΔH) in the Hansen solubility parameter.

**[Table 2]**

| No. | Solvent name | CAS No. | van der Waals volume (Å³) | Dipole moment (D) | Dipole moment per van der Waals volume (D/Å³) | ΔH |
|---|---|---|---|---|---|---|
| 26 | benzyl alcohol | 100-51-6 | 65.7 | 1.84 | 0.028 | 12.5 |
| 27 | methyl pyruvate | 600-22-6 | 55.3 | 1.13 | 0.020 | 8.9 |
| 28 | 4-methyl-2-pentanol | 108-11-2 | 72.3 | 2.28 | 0.032 | 9.9 |
| 29 | 4-methyl-2-pentanone | 108-10-1 | 69.4 | 3.34 | 0.048 | 3.9 |
| 30 | ethyl lactate | 97-64-3 | 67.8 | 2.56 | 0.038 | 13.1 |
| 31 | 1-methoxy-2-propanol | 107-98-2 | 57.1 | 2.43 | 0.043 | 12.5 |
| 32 | propylene glycol 1-monomethyl ether 2-acetate | 108-65-6 | 77.4 | 3.99 | 0.052 | 6.6 |
| 33 | 2,6-dimethylpyridine | 108-48-5 | 68.5 | 1.21 | 0.018 | 4.7 |
| 34 | isopropyl alcohol | 67-63-0 | 43.5 | 2.05 | 0.047 | 12.8 |
| 35 | ethanol | 64-17-5 | 34 | 1.91 | 0.056 | 17.2 |
| 36 | acetophenone | 98-86-2 | 72.4 | 3.46 | 0.048 | 4.5 |
| 37 | 2-(hydroxymethyl)furan | 98-00-0 | 53.5 | 1.63 | 0.030 | 14.4 |
| 38 | 1,4-dioxane | 123-91-1 | 49.7 | 0.002 | 0.000 | 8.1 |
| 39 | toluene | 108-88-3 | 61.7 | 0.682 | 0.011 | 3.3 |
| 40 | tert-butyl methyl ether | 1634-04-4 | 72.7 | 1.716 | 0.024 | 3.2 |
| 41 | tetrahydrofuran | 109-99-9 | 45.7 | 2.285 | 0.050 | 5.8 |
| 42 | water | 7732-18-5 | 14.8 | 2.07 | 0.140 | 42.3 |
| 43 | ethylene glycol | 107-21-1 | 37.9 | 0.942 | 0.025 | 27.4 |

As a solvent having a dipole moment (D) per van der Waals volume (Å³) of less than 0.055 D/Å³ or a hydrogen-bonding term (ΔH) in the Hansen solubility parameter of more than 17 MPa^{1/2}, the second solvent used in the present embodiment may be, for example, a ketone solvent, an ether solvent, an ester solvent, an alcohol solvent, a hydrocarbon solvent, an aromatic hydrocarbon solvent, a heterocyclic solvent, a halogen solvent, water, or the like that satisfies the above requirements.

The ketone solvent that can be used as the second solvent is, for example, methyl ethyl ketone, 4-methyl-2-pentanone (methyl isobutyl ketone), di-n-butyl ketone, acetophenone, methyl pyruvate, or the like.
The ether solvent that can be used as the second solvent is, for example, a chain ether, such as 1-methoxy-2-propanol, diethyl ether, isopropyl ether, t-butyl methyl ether, or anisole; a cyclic ether, such as 1,4-dioxane, tetrahydrofuran, or 1,3-dioxolane; or the like.
The ester solvent that can be used as the second solvent is, for example, ethanol, propylene glycol-1-monomethyl ether-2-acetate, propylene glycol-1-monoethyl ether-2-acetate, propylene glycol-1-monobutyl ether-2-acetate, ethylene glycol diacetate, diethyl oxalate, methyl 3-methoxypropionate, or the like.
The alcohol solvent that can be used as the second solvent is, for example, isopropyl alcohol, t-butyl alcohol, 4-methyl-pentanol, benzyl alcohol, ethylene glycol, propylene glycol, ethyl lactate, butyl lactate, 2-(hydroxymethyl)furan, or the like.

The hydrocarbon solvent that can be used as the second solvent is, for example, a chain hydrocarbon solvent, such as n-pentane, n-hexane, n-heptane, or n-octane; a cyclic hydrocarbon solvent, such as cyclopentane, cyclohexane, methylcyclohexane, ethylcyclohexane, butylcyclohexane, cycloheptane, or cyclooctane; or the like.
The aromatic hydrocarbon solvent that can be used as the second solvent is, for example, benzene, toluene, xylene, ethylbenzene, mesitylene, or the like.
The heterocyclic solvent that can be used as the second solvent is, for example, pyridine, picoline, 2,6-dimethylpyridine, quinoline, 1-methylpyrrole, 3-methylpyrrole, or the like. The halogen solvent that can be used as the second solvent is, for example, dichloromethane, chloroform, 1,2-dichloroethane, chlorobenzene, or the like.

### [2. Method for Producing Solvate of Polyacid Salt]

The method for producing a solvate of a polyacid salt may be, but is not limited to, a known and commonly used method for producing a solvate.

The step of producing a solvate of a polyacid salt may include (1) a step of dissolving or dispersing the polyacid salt in one or more first solvents that are good solvents for the polyacid salt to prepare a good solvent solution, (2) a step of distilling off the solvent(s) from the good solvent solution at atmospheric pressure or under reduced pressure, (2') a step of cooling the good solvent solution from the temperature at the time of dissolving the polyacid salt, (2") a step of adding a second solvent other than the first solvent that is a poor solvent for the polyacid salt to the good solvent solution, (2"') a step of adding the second solvent to the good solvent solution, then cooling the good solvent solution, and the like, and when a deposit or precipitate forms, (3) a step of filtering off the solid, (4) a step of drying the filtered solid, and the like.

The method for producing a solvate of a polyacid salt according to the present embodiment is, for example, a method for producing a solvate of a polyacid salt including a step of dissolving or dispersing a polyacid salt in one or more first solvents that are good solvents for the polyacid salt to prepare a good solvent solution.

A method for producing a solvate of a polyacid salt according to another embodiment is, for example, a method for producing a solvate of a polyacid salt including a step of dissolving a polyacid salt in one or more first solvents that are good solvents for the polyacid salt to prepare a good solvent solution and a step of adding a second solvent that is a poor solvent for the polyacid salt to the good solvent solution to precipitate a solvate of the polyacid salt.

The solvate of the polyacid salt includes not only a solid (including a powder or the like) or an oily substance deposited or precipitated through these production steps, or a crude, but also a substance produced by concentrating the good solvent solution or a saturated solution thereof, and these may be used as they are.

### [3. Film-Forming Material]

A film-forming material according to the present embodiment is produced using a solvate of a polyacid salt.

The solvate of the polyacid salt according to the present embodiment has improved dispersibility or solubility in a solvent and can be suitably used as a film-forming material.

The film-forming material may be produced, for example, by dissolving the polyacid salt in one or more first solvents that are good solvents for the polyacid salt, concentrating or saturating the resulting solution to bring the polyacid salt into a state of being solvated with the first solvent, stocking the resulting solution, and then dissolving the resulting solution in the first solvent and/or the second solvent at the time of producing the film-forming material.

The film-forming material may also be produced by, for example, precipitating a solvate of a polyacid salt through a step of dissolving the polyacid salt in one or more first solvents that are good solvents for the polyacid salt to prepare a good solvent solution, and then dissolving the prepared solvate of the polyacid salt in the first solvent and/or the second solvent.

The film-forming material may also be produced, for example, by dissolving a solvate of a polyacid salt in a solid state (including a powder, a crystal, or the like) in the first solvent and/or the second solvent at the time of producing the film-forming material, the solvate having been precipitated through a step of dissolving the polyacid salt in one or more first solvents that are good solvents for the polyacid salt to prepare a good solvent solution and a step of adding a solvent that is a poor solvent for the polyacid salt to the good solvent solution to precipitate the solvate of the polyacid salt.

A film-forming material according to another embodiment may contain a solvate of a polyacid salt and a first solvent. From the perspective of allowing a polyacid salt to be contained at a higher concentration, the first solvent can be a solvent having a dipole moment (D) per van der Waals volume (Å³) of 0.055 D/Å³ or more and a hydrogen-bonding term (ΔH) in the Hansen solubility parameter of 17 or less.

The film-forming material according to the present embodiment has improved solubility or dispersibility in a solvent other than the first solvent by using the solvate of the polyacid salt and may contain the solvate of the polyacid salt and a solvent classified as the second solvent.
The second solvent may be one of the solvents exemplified in Table 2, for example, ethyl lactate, propylene glycol-1-monomethyl ether-2-acetate, isopropyl alcohol, or the like.

### EXAMPLES

The present invention will be more specifically described below with reference to examples, but the present invention is not limited to these examples.

### [1. Synthesis of Polyacid Salt]

Polyacid salts 1 to 7 were synthesized by the following methods.

### <Polyacid salt 1: hexakis(phenylbis(2-(trifluoromethyl)phenyl)sulfonium)metatungstate>

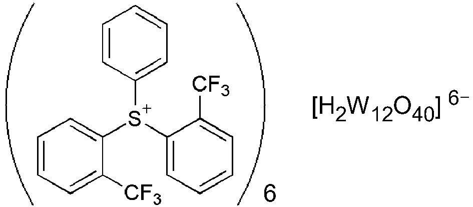

6.68 g of phenylbis(2-(trifluoromethyl)phenyl)sulfonium chloride was dissolved in 99.36 g of pure water, 6.88 g of ammonium metatungstate (manufactured by Nippon Inorganic Colour & Chemical Co., Ltd.: ammonium metatungstate hydrate ((NH₄)₆[H₂W₁₂O₄₀]·xH₂O)) was added thereto, and the reaction liquid was stirred at room temperature for 30 minutes. A powder produced by filtering the reaction liquid was dried under reduced pressure at room temperature for 18 hours. The dried powder was dissolved in 100 g of dimethylformamide, 335 g of methanol was then added thereto, and a target compound (polyacid salt 1) was produced by crystallization at room temperature.
¹H-NMR (400MHz, DMSO-D6): δ (ppm) = 8.35-7.65 (m, 13H), 5.97 (s, 0.33H).
¹⁸³W-NMR (20.84MHz, DMSO-D6): δ(ppm) = -100.13 (s, 12W).
ESI-MS: POSITIVE m/z 399.1 ([C₂₀H₁₃F₆S]⁺)
   NEGATIVE m/z 712.3 (median) ([H₄W₁₂O₄₀]⁴⁻)

### <Polyacid salt 2: metatungstate containing five phenylbis(2-(trifluoromethyl)phenyl)sulfonium cations (the following compound)

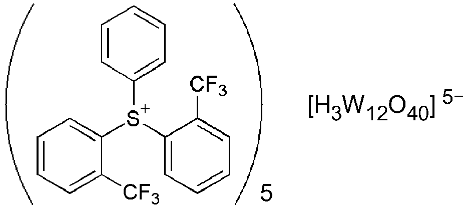

100 g of 0.1 mol/L aqueous hydrochloric acid were added to 16.67 g of ammonium metatungstate (manufactured by Nippon Inorganic Colour & Chemical Co., Ltd.: ammonium metatungstate hydrate ((NH₄)₆[H₂W₁₂O₄₀]·xH₂O) and were stirred at room temperature for 60 minutes. An aqueous solution of 13.47 g of phenyl bis(2-(trifluoromethyl)phenyl)sulfonium chloride in 200 g of pure water was then added to this reaction liquid and was stirred at room temperature for 30 minutes. A powder prepared by filtering the reaction liquid was dried under reduced pressure at room temperature for 18 hours. The dried powder was dissolved in 100 g of dimethylformamide, 335 g of methanol was then added thereto, and a target compound (polyacid salt 2) was produced by crystallization at room temperature.
¹H-NMR (400MHz, DMSO-D6): δ (ppm) = 8.35-7.65 (m, 13H), 6.77 (s, 0.60H).
¹⁸³W-NMR (20.84MHz, DMSO-D6): δ (ppm) = -87.59 (s, 12W).
ESI-MS: POSITIVE m/z 399.1 ([C₂₀H₁₃F₆S]⁺)
   NEGATIVE m/z 712.3 (median) ([H₄W₁₂O₄₀]⁴⁻)

### <Polyacid salt 3: tetrakis(phenylbis(2-(trifluoromethyl)phenyl)sulfonium)silicotungstate>

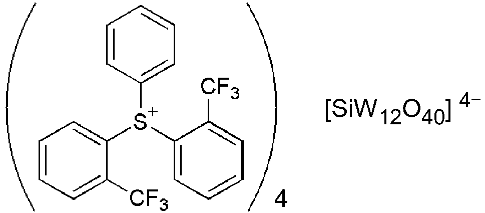

37.26 g of phenylbis(2-(trifluoromethyl)phenyl)sulfonium chloride was dissolved in 50 g of cyclohexanone, 41.06 g of silicotungstic acid (H₄[SiW₁₂O₄₀]·26H₂O) was added thereto, and the reaction liquid was stirred at room temperature for 2 hours. A powder produced by filtering the reaction liquid was dried under reduced pressure at room temperature for 18 hours. The dried powder was crystallized at room temperature using dichloromethane and acetonitrile to produce a target compound (polyacid salt 3).
¹H-NMR (400MHz, DMSO-D6): δ (ppm) = 8.34-7.63 (m, 52H).
¹⁸³W-NMR (20.84MHz, DMSO-D6): δ (ppm) = -92.7 (s, 12W).
ESI-MS:NEGATIVE m/z 718.5 (median) ([SiW₁₂O₄₀]⁴⁻)
XPS analysis S:W (the mole ratio of S to W) = 4:12

### <Polyacid salt 4: tris(phenylbis(2-(trifluoromethyl)phenyl)sulfonium)phosphotungstate>

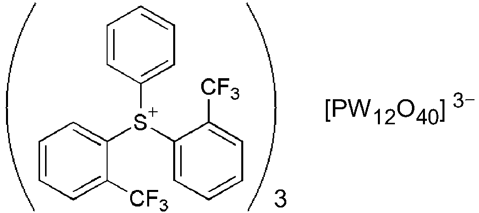

A target compound (polyacid salt 4) was produced in the same manner as in the method for synthesizing the polyacid salt 3 except that, as raw material compounds, 37.26 g of phenylbis(2-(trifluoromethyl)phenyl)sulfonium chloride was changed to 27.95 g and the silicotungstic acid was replaced by 41.97 g of phosphotungstic acid (H₃[PW₁₂O₄₀]·30H₂O).
¹H-NMR (400MHz, DMSO-D6): δ (ppm) = 8.36-7.63 (m, 39H).
¹⁸³W-NMR (20.84MHz, DMSO-D6): δ (ppm) = -86.7 (s, 12W).
ESI-MS: NEGATIVE m/z 959 (median) ([PW₁₂O₄₀]³⁻)
XPS analysis S:W (the mole ratio of S to W) = 3:12

### <Polyacid salt 5: tris((2-hydroxyethyl)trimethylammonium)phosphotungstate>

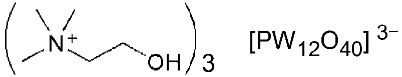

A target compound (polyacid salt 5) was produced in the same manner as in the method for synthesizing the polyacid salt 3 except that, as raw material compounds, phenylbis(2-(trifluoromethyl)phenyl)sulfonium chloride was replaced by 0.2 g of (2-hydroxyethyl)trimethylammonium chloride and the silicotungstic acid was replaced by 1.0 g of phosphotungstic acid (H₃[PW₁₂O₄₀]·30H₂O).
¹H-NMR (400MHz, DMSO-D6): δ (ppm) = 5.28(br, 3H), 3.84(m, 6H), 3.40(m, 6H), 3.11 (s, 27H).
¹⁸³W-NMR (20.84MHz, DMSO-D6): δ (ppm) = -86.7(s, 12W).
ESI-MS: NEGATIVE m/z 959 (median) ([PW₁₂O₄₀]³⁻)
XPS analysis N:W (the mole ratio of N to W) = 3:12

### <Polyacid salt 6: tris(tetrahexylammonium)phosphotungstate)>

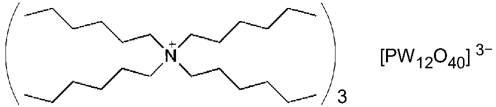

A target compound (polyacid salt 6) was produced in the same manner as in the method for synthesizing the polyacid salt 3 except that, as raw material compounds, phenylbis(2-(trifluoromethyl)phenyl)sulfonium chloride was replaced by 0.5 g of tetrahexylammonium iodide and the silicotungstic acid was replaced by 1.0 g of phosphotungstic acid (H₃[PW₁₂O₄₀]·30H₂O).
¹H-NMR (400MHz, DMSO-D6): δ (ppm)=3.16 (m, 24H), 1.57 (m, 24H), 1.30 (s, 72H), 0.88 (t, 36H).
¹⁸³W-NMR (20.84MHz, DMSO-D6): δ (ppm) = -86.7 (s, 12W).
ESI-MS: NEGATIVE m/z 959 (median) ([PW₁₂O₄₀]³⁻)
XPS analysis N:W (the mole ratio of N to W) = 3:12

### <Polyacid salt 7: tris(tetraphenylphosphonium)phosphotungstate>

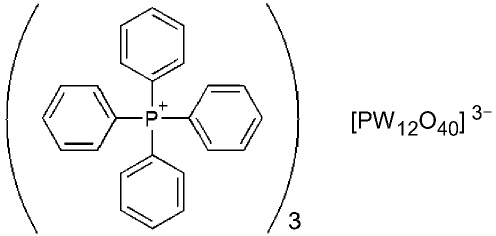

A target compound (polyacid salt 7) was produced in the same manner as in the method for synthesizing the polyacid salt 3 except that, as raw material compounds, phenylbis(2-(trifluoromethyl)phenyl)sulfonium chloride was replaced by 0.45 g of tetraphenylphosphonium chloride and the silicotungstic acid was replaced by 1.0 g of phosphotungstic acid(H₃[PW₁₂O₄₀]·30H₂O).
¹H-NMR (400MHz, DMSO-D6): δ (ppm) = 8.05-7.65 (m, 60H).
¹⁸³W-NMR (20.84MHz, DMSO-D6): δ (ppm) = -86.7(s, 12W).
ESI-MS: NEGATIVE m/z 959 (median) ([PW₁₂O₄₀]³⁻)
XPS analysis P:W(the mole ratio of P to W) = 4:12

### [2. Synthesis of Solvate of Polyacid Salt]

As solvates of the polyacid salts 1 to 4, solvates 1 to 9 of polyacid salts were synthesized by the following methods.

### <Solvate 1 of polyacid salt: solvate of hexakis(phenylbis(2-(trifluoromethyl)phenyl)sulfonium)metatungstate solvated with 4-hydroxy-2-butanone and t-butyl methyl ether>

10 g of the polyacid salt 1 (hexakis(phenylbis(2-(trifluoromethyl)phenyl)sulfonium)metatungstate) was dissolved in 100 g of 4-hydroxy-2-butanone. 1000 g of t-butyl methyl ether was added thereto and was stirred for 5 minutes. The resulting precipitate was filtered off with suction and was dried under reduced pressure for 10 minutes.

¹H-NMR (400MHz, DMSO-D6): δ (ppm) = 8.40-7.60 (m, 13H), 5.97 (s, 0.31H), 4.55 (br, 0.026H), 3.63 (m, 0.051H), 3.08 (s, 0.014H), 2.53 (m, 0.051H), 2.09 (s, 0.077H), 1.11 (s, 0.042H).

### <Solvate 2 of polyacid salt: solvate of hexakis(phenylbis(2-(trifluoromethyl)phenyl)sulfonium)metatungstate solvated with 2-cyclopenten-1-one, t-butyl methyl ether, and p-menthane>

2 g of the polyacid salt 1 (hexakis(phenylbis(2-(trifluoromethyl)phenyl)sulfonium)metatungstate) was dissolved in 8 g of 2-cyclopenten-1-one to prepare a 20% by weight solution. 60 g of p-menthane and 100 g of t-butyl methyl ether were added thereto in this order. The resulting precipitate was filtered off with suction and was dried under reduced pressure for 10 minutes.

¹H-NMR (400MHz, DMSO-D6): δ (ppm) = 8.40-7.60 (m, 80H), 6.0 (s, 2H), 3.08 (s, 3H), 2.65 (m, 4H), 2.25 (m, 4.0H), 1.11 (s, 9.0H), 0.09-0.08 (m, 5.2H).

### <Solvate 3 of polyacid salt: solvate of polyacid salt 2 solvated with 4-hydroxy-2-butanone and t-butyl methyl ether>

10 g of the synthesized polyacid salt 2 was weighed and dissolved in 90 g of 4-hydroxy-2-butanone. 1000 g of t-butyl methyl ether was added thereto and was stirred for 5 minutes. The resulting precipitate was filtered off with suction and was dried under reduced pressure for 10 minutes.

¹H-NMR (400MHz, DMSO-D6): δ (ppm) = 8.40-7.60 (m, 65H), 6.71 (s, 3H), 4.55 (br, 3H), 3.63 (t, 5.1H), 3.08 (s, 1.2H), 2.53 (m, 4.9H), 2.09 (s, 7.0H), 1.11 (s, 4H).

### <Solvate 4 of polyacid salt: solvate of polyacid salt 2 solvated with 2-cyclopenten-1-one, p-menthane, and t-butyl methyl ether>

2 g of the synthesized polyacid salt 2 was weighed and dissolved in 8 g of 2-cyclopenten-1-one. 60 g of p-menthane and 600 g of t-butyl methyl ether were added thereto in this order. The resulting precipitate was filtered off with suction and was dried for 10 minutes. ¹H-NMR (400MHz, DMSO-D6): δ (ppm) = 8.40-7.60 (m, 67H), 6.71 (s, 3H), 6.17 (s, 2H), 3.08 (s, 2H), 2.65 (m, 4H), 2.25 (m, 4.0H), 1.11(s, 6.0H), 0.08-0.09 (m, 4.7H).

### <Solvate 5 of polyacid salt: solvate of tetrakis(phenylbis(2-(trifluoromethyl)phenyl)sulfonium)silicotungstate solvated with 4-hydroxy-2-butanone>

2 g of the polyacid salt 3 (tetrakis(phenylbis(2-(trifluoromethyl)phenyl)sulfonium)silicotungstate) was dissolved in 8 g of 4-hydroxy-2-butanone to prepare a 20% by weight solution. 1000 g of t-butyl methyl ether was added thereto. The resulting precipitate was filtered off with suction and was dried under reduced pressure for 10 minutes.

¹H-NMR (400MHz, DMSO-D6): δ (ppm) = 8.40-7.60 (m, 1H), 4.55 (br, 0.038H), 3.63 (m, 0.077H), 2.53 (m, 0.077H), 2.09 (s, 0.12H).

### <Solvate 6 of polyacid salt: solvate of tetrakis(phenylbis(2-(trifluoromethyl)phenyl)sulfonium)silicotungstate solvated with 2-cyclopenten-1-one>

2 g of the polyacid salt 3 (tetrakis(phenylbis(2-(trifluoromethyl)phenyl)sulfonium)silicotungstate) was dissolved in 8 g of 2-cyclopenten-1-one to prepare a 20% by weight solution. 1000 g of t-butyl methyl ether was added thereto. The resulting precipitate was filtered off with suction and was dried under reduced pressure for 10 minutes.

¹H-NMR (400MHz, DMSO-D6): δ (ppm) = 8.40-7.60 (m, 1H), 6.17 (s, 0.038H), 2.65 (m, 0.077H), 2.25 (m, 0.077H).

### <Solvate 8 of polyacid salt: solvate of tris(phenylbis(2-(trifluoromethyl)phenyl)sulfonium)phosphotungstate solvated with 2-cyclopenten-1-one>

2 g of the polyacid salt 4 (tris(phenylbis(2-(trifluoromethyl)phenyl)sulfonium)phosphotungstate) was dissolved in 18 g of 2-cyclopenten-1-one to prepare a 10% by weight solution. 100 g of p-menthane and 100 g of t-butyl methyl ether were added thereto in this order. The resulting precipitate was filtered off with suction and was dried under reduced pressure for 10 minutes.

¹H-NMR (400MHz, DMSO-D6): δ (ppm) = 8.40-7.60 (m, 1H), 6.17 (s, 0.05H), 2.65 (m, 1.0H), 2.25 (m, 1.0H).

### <Solvate 7 of polyacid salt: solvate of tris(phenylbis(2-(trifluoromethyl)phenyl)sulfonium)phosphotungstate solvated with 4-hydroxy-2-butanone>

2 g of the polyacid salt 4 (tris(phenylbis(2-(trifluoromethyl)phenyl)sulfonium)phosphotungstate) was dissolved in 8 g of 4-hydroxy-2-butanone to prepare a 20% by weight solution. 1000 g of t-butyl methyl ether was added thereto. The resulting precipitate was filtered off with suction and was dried under reduced pressure for 10 minutes.

¹H-NMR (400MHz, DMSO-D6): δ (ppm) = 8.40-7.60 (m, 1H), 4.55 (br, 0.05H), 3.63 (t, 0.1H), 2.53 (m, 0.1H), 2.09 (s, 0.15H).

### <Solvate 9 of polyacid salt: solvate of tris(phenylbis(2-(trifluoromethyl)phenyl)sulfonium)phosphotungstate solvated with cyclohexanone and p-menthane>

2 g of the polyacid salt 4 (tris(phenylbis(2-(trifluoromethyl)phenyl)sulfonium)phosphotungstate) was dissolved in 18 g of cyclohexanone to prepare a 10% by weight solution. 200 g of p-menthane and 200 g of t-butyl methyl ether were added thereto in this order. The resulting precipitate was filtered off with suction and was dried under reduced pressure for 10 minutes.

¹H-NMR (400MHz, DMSO-D6): δ (ppm) = 8.40-7.60 (m, 1H), 2.25 (m, 0.06H), 1.77 (m, 0.06H), 1.66 (m, 0.03H), 1.50-1.2 (m, 0.04H), 1.0-0.8 (m, 0.08H).

### [3. Solubility Test and Evaluation]

The polyacid salts 1 to 4 synthesized above were tested for their solubility in various solvents listed in Table 3 by the following method.

First, 1 g of powder of each of the polyacid salts 1 to 4 was weighed in a beaker. The solvent listed in Table 3 was then added, the mass concentration (% by weight) was calculated from the addition amount of the solvent at the time when dissolution was visually confirmed, and the solubility was evaluated according to the following criteria. Table 3 shows the results.
A: dissolved at 1% by weight or more
B: dissolved at 0.1% by weight or more and less than 1% by weight
C: not dissolved at less than 0.1% by weight

**[Table 3]**

| No. | Solvent | Polyacid salt 1 | Polyacid salt 2 | Polyacid salt 3 | Polyacid salt 4 |
|---|---|---|---|---|---|
| 1 | cyclohexanone | C | C | A | A |
| 2 | cyclopentanone | C | C | A | A |
| 3 | 2-cyclohexen-1-one | C | C | A | A |
| 4 | 3-methyl-2-cyclopentenone | C | C | A | A |
| 5 | 4-hydroxy-2-butanone | A | A (10%) | A | A |
| 6 | ethyl 2-hydroxypropionate | C | C | C | C |
| 7 | 1-methoxy-2-propanol | C | C | C | C |
| 8 | propylene glycol-1-monomethyl ether-2-acetate | C | C | C | C |
| 9 | diacetone alcohol | C | C | A | A |

For the solvates 1 to 6, 8, and 9 of the polyacid salts synthesized above, the solubility in various solvents listed in Table 4 was evaluated using the same test method and criteria as described above. Table 4 shows the results.

**[Table 4]**

| N o. | Solvent | Solvate of polyacid salt 1 | | Solvate of polyacid salt 2 | | Solvate of polyacid salt 3 | | Solvate of polyacid salt 4 | |
|---|---|---|---|---|---|---|---|---|---|
| | | Solvate 1 | Solvate 2 | Solvate 3 | Solvate 4 | Solvate 5 | Solvate 6 | Solvate 8 | Solvate 9 |
| 1 | cyclohexanone | A | A | A | A | A | A | A | A |
| 2 | cyclopentanone | A | A | A | A | A | A | A | A |
| 3 | 2-cyclohexen-1-one | A | A | A | A | A | A | A | A |
| 4 | 3-methyl-2-cyclopentenone | A | A | A | A | A | A | A | A |
| 5 | 4-hydroxy-2-butanone | A | A | A (30%) | A (30%) | A | A | A | A |
| 6 | ethyl 2-hydroxypropionate | B | B | B | A | A | A | A | A |
| 7 | 1-methoxy-2-propanol | C | C | C | C | A | A | A | A |
| 8 | propylene glycol-1-monomethyl ether-2-acetate | C | C | C | C | A | A | A | A |
| 9 | diacetone alcohol | A | A | A | A | A | A | A | A |

The results of Tables 3 and 4 show that, all of the polyacid salts 1 to 4 solvated with a good solvent or with a good solvent and a poor solvent have improved solubility in various solvents.
More specifically, for example, as shown in Table 3, although the polyacid salt 2 itself was dissolved in 4-hydroxy-2-butanone of No. 5 at approximately 10% by weight, the solvates (solvates 3 and 4) of the polyacid salt 2 were dissolved in 4-hydroxy-2-butanone at approximately 30% by weight, thus exhibiting greatly improved solubility. Such an effect of improving the solubility was similarly observed in the solubility of other polyacid salts and solvates of polyacid salts in various solvents.

Furthermore, also as can be seen from Table 3, the polyacid salts 1 and 2 themselves were evaluated as C for the solubility in the solvents of Nos. 1 to 4 and Nos. 6 to 9 except 4-hydroxy-2-butanone of No. 5. **In** contrast, when the polyacid salts 1 and 2 were converted into solvates (solvates 1 to 4), the evaluation of solubility in the solvents of Nos. 1 to 4 and 9 changed from C to A, and the evaluation of solubility in the solvent of No. 6 changed from C to B or A, thereby improving the solubility. These results show that the solvates 1 to 4 of the polyacid salts 1 and 2 can widen the range of choices of the solvent that can be used for the polyacid salts 1 and 2.
With respect to the polyacid salts 3 and 4 as well, the polyacid salts 3 and 4 themselves were rated C in the evaluation of solubility in the solvents of Nos. 6 to 8; however, when converted into solvates (solvates 5, 6, 8, and 9), the rating changed from C to A in the solvents of Nos. 6 to 8, thereby improving the solubility in all cases.

## Claims

1. A solvate of a polyacid salt, wherein
a solvent that solvates the polyacid salt includes a first solvent that is a good solvent for the polyacid salt.

2. The solvate of the polyacid salt according to claim 1, wherein an anion moiety of the polyacid salt is an isopolyoxomolybdate anion, an isopolyoxotungstate anion, an isopolyoxoniobate anion, an isopolyoxotantalate anion, a heteropolyoxomolybdate anion, a heteropolyoxotungstate anion, a heteropolyoxovanadate anion, a heteropolyoxoniobate anion, or a heteropolyoxotantalate anion.

3. The solvate of the polyacid salt according to claim 1, wherein a cation moiety of the polyacid salt is an onium cation.

4. The solvate of the polyacid salt according to claim 3, wherein the onium cation is an organic sulfonium cation, an organic iodonium cation, an organic quaternary ammonium cation, or an organic phosphonium cation.

5. The solvate of the polyacid salt according to claim 1, wherein the first solvent has a dipole moment (D) per van der Waals volume (Å³) of 0.055 D/Å³ or more and a hydrogen-bonding term (ΔH) in a Hansen solubility parameter of 17 MPa^{1/2} or less.

6. The solvate of the polyacid salt according to claim 1, wherein the solvent that solvates the polyacid salt further includes a second solvent other than the first solvent, the second solvent being a poor solvent for the polyacid salt.

7. A method for producing a solvate of a polyacid salt, the method comprising
dissolving the polyacid salt in one or more first solvents that are good solvents for the polyacid salt to prepare a good solvent solution.

8. The method for producing a solvate of a polyacid salt according to claim 7, further comprising adding a second solvent other than the first solvent to the good solvent solution, the second solvent being a poor solvent for the polyacid salt.

9. A film-forming material produced using the solvate of the polyacid salt according to claim 1.
